# EUROPÄISCHE PATENTSCHRIFT

(11) **EP 3 787 784 B1**
(45) Veröffentlichungstag und Bekanntmachung des Hinweises auf die Patenterteilung: **07.06.2023**
(21) Anmeldenummer: 19725649.8
(22) Anmeldetag: 29.04.2019
(51) Int. Cl.: C12M 1/24, C12M 1/00, B01L 3/00, B01L 9/06, F21V 33/00

(54) **VORRICHTUNG ZUM BELEUCHTEN VON SUSPENSIONSZELLKULTUREN**
DEVICE FOR ILLUMINATING SUSPENSION CELL CULTURES
DISPOSITIF POUR ÉCLAIRER DES CULTURES CELLULAIRES EN SUSPENSION

(30) Priorität: 30.04.2018 DE 102018110329
(43) Veröffentlichungstag der Anmeldung: 10.03.2021
(73) Patentinhaber: Fraunhofer-Gesellschaft zur Förderung der angewandten Forschung e.V., 80686 München (DE)
(72) Erfinder: RASCHE, Stefan, 41812 Erkelenz (DE); SCHUBERT, Max, 52062 Aachen (DE); BEUEL, Ann-Katrin, 52074 Aachen (DE)
(74) Vertreter: Schiweck Weinzierl Koch Patentanwälte Partnerschaft mbB
(86) Internationale Anmeldenummer: PCT/EP2019/060889
(87) Internationale Veröffentlichungsnummer: WO 2019/211227

(56) Entgegenhaltungen:
- WO-A1-2017/157404
- DE-U1-202007 013 405
- DE-U1-202007 013 405
- DE-U1-202010 006 193
- JP-A- H11 262 336
- US-A1- 2014 273 197

## Beschreibung

### Gebiet der Erfindung

Die Erfindung betrifft eine Vorrichtung zum Beleuchten von Suspensionskulturen, welche bevorzugt in einem Schüttelinkubator zum Einsatz kommen können zum Bereitstellen mehrerer unterschiedlicher Lichtbedingungen in einem Schüttelinkubator.

### Stand der Technik

Im Bereich der Pflanzen-Biotechnologie werden unter anderem pflanzliche Suspensionszellkulturen oder Algen dazu verwendet, bestimmte wertvolle Inhaltstoffe zu produzieren, z.B. sekundäre Pflanzenstoffe, Zucker, Öle und Fette usw. Ein Faktor, der für eine erfolgreiche Kultivierung maßgeblich ist, ist die Auswahl geeigneter Lichtbedingungen. Dabei ist Licht nicht nur eine entscheidende Größe für die Photosynthese-Aktivität in phototropen Organismen. Über seine Bedingungen wie Intensität, Dauer und/oder Spektrum kann der Stoffwechsel bei phototropen wie auch nicht-phototropen Kulturen über deren spezielle Photorezeptoren beeinflusst und so die Produktion von Biomasse oder sekundären Pflanzenstoffen signifikant beeinflusst werden. Dabei ist jedoch zu berücksichtigen, dass es keine universellen Bedingungen gibt, die den gleichen Effekt bei verschiedenen pflanzlichen Zellkulturen oder Algen verursachen. Vielmehr müssen die Bedingungen für jede einzelne Kultur und im Hinblick auf das gewünschte Ergebnis (z.B. mehr Biomasse und/oder gesteigerte Produktion eines spezifischen sekundären Pflanzenstoffs) individuell bestimmt werden.

Pflanzliche Suspensionskulturen, wie auch Algen, werden nicht statisch, sondern submers in einem geeigneten Nährmedium kultiviert. Dabei ist es für eine erfolgreiche Kultivierung unabdingbar, dass die Kulturen konstant durchmischt werden, was üblicherweise mittels eines temperierbaren Schüttelinkubators realisiert wird. Problematisch hierbei ist jedoch, dass pro Schüttelinkubator zwar mehrere verschiedene Kulturen parallel angezogen werden können, jedoch in einem Schüttelinkubator nur eine Lichtbedingung realisiert werden kann. Dieser Umstand mach eine experimentelle Optimierung einer Kultur in Bezug auf verschiedene Lichteinstellung extrem langwierig bis wirtschaftlich unmöglich, da die Lichtbedingungen in einem Schüttelinkubator nur sequentiell, also jeweils erst bei der Anzucht der nächsten Kulturen verändert werden können.

Eine gängige Möglichkeit, verschiedene Lichtbedingungen parallel zu realisieren, besteht darin, mehrere Schütteinkubatoren zu verwenden. Dabei stehen je nach Hersteller verschiedene Möglichkeiten der Beleuchtung zur Verfügung, bei der die Leuchtmittel an der Decke des Inkubators angebracht sind. Es sind beispielsweise Schüttelinkubatoren auf dem Markt erhältlich, welche neben üblichen Fluoreszenzlampen auch mit LED-Modulen bestückt sind und eine Einstellung der Intensität und der Dauer der Beleuchtung ermöglichen. Des Weiteren sind auch Schüttelinkubatoren erhältlich, in denen spezielle LED-Module zum Einsatz kommen, die neben Dauer und Intensität auch eine Auswahl des Spektralbereichs des abgestrahlten Lichts erlauben. Beide Lösungen haben jedoch gemein, dass alle Beleuchtungseinstellungen für ein Inkubationssegment als Ganzes gelten. Sollen mehrere verschiedene Beleuchtungsbedingungen parallel realisiert werden, muss die Anzahl der Inkubatoren entsprechend erhöht werden, was mit deutlichen Kosten und einem erhöhten Platzbedarf einhergeht. Das Dokument DE202007013405U zeigt eine Beleuchtungseinrichtung für Schüttelkolben in Inkubatoren mit einem flächigen Träger, dessen eine Seite mit mehreren über den Träger verteilten Leuchtdioden versehen ist, wobei der Träger in eine Gefäßhalterung integriert ist, welche dazu ausgelegt ist, z.B. einen Schüttelkolben so zu haltern, dass der Boden des Schüttelkolbens der mit Leuchtdioden versehenen Seite des Trägers zugewandt ist.

Es ist daher die Aufgabe der vorliegenden Erfindung eine Vorrichtung bereitzustellen, mittels welcher die Anzucht von verschiedenen Kulturen bei individuellen Beleuchtungsbedingungen in einem Schüttelinkubator ermöglicht wird.

### Zusammenfassung der Erfindung

Gelöst wird diese Aufgabe durch eine Vorrichtung zum Beleuchten von Suspensionszellkulturen gemäß Anspruch 1. Vorteilhafte Ausführungsformen der erfindungsgemäßen Vorrichtung finden sich in den abhängigen Ansprüchen. Insbesondere löst die vorliegende Erfindung die Aufgabe, indem sie eine kompakte, lichtdichte und individuell beleuchtbare Einheit bereitstellt, von denen eine Mehrzahl in jedem Schüttelinkubator installiert werden kann, um so in einem einzigen Schüttelinkubator unterschiedliche Lichtbedingungen für die Anzucht von Zellkulturen bereitzustellen.

In verschiedenen Ausführungsformen wird eine Vorrichtung zum Beleuchten von Suspensionszellkulturen bereitgestellt (nachfolgend als Vorrichtung bezeichnet) mit einer Beleuchtungseinheit, welche mindestens zwei Leuchtmittel aufweist und zur Aufnahme eines Behälters mit der sich darin befindenden Suspensionszellkulturen eingerichtet ist, und einer Abschirmeinrichtung, welche eingerichtet ist, mindestens lateral eine Kontamination der Umgebung der Vorrichtung mit Streulicht zu verhindern. Bei dem Behälter kann es sich um ein Kultur oder ein Kultivierungsgefäß handeln.

Die Beleuchtungseinheit stellt im Prinzip die Basis der Vorrichtung dar und kann ein Gehäuse aufweisen, beispielsweise aus einem Kunststoff, in dem die mindestens zwei Leuchtmittel angeordnet sind sowie auch weitere Elemente, die für den Betrieb der Vorrichtung erforderlich sind. Zur Verwendung der Vorrichtung kann der die Suspensionszellkultur aufweisende Behälter in die Beleuchtungseinheit gestellt werden, wobei die Kontaktfläche des Bodens des Behälters mit der Beleuchtungseinheit in einer Vertiefung in der Beleuchtungseinheit angeordnet ist.

Die Leuchtmittel können bevorzugt in der Vertiefung so angeordnet sein, dass sie den unteren Teil des Behälters anleuchten.

Um zu verhindern, dass das von den Leuchtmitteln abgestrahlte Licht aus der Vorrichtung austritt und eventuell auf benachbart angeordnete Zellkulturen einwirkt, ist die Abschirmeinrichtung vorgesehen, welche eine lichtdichte Barriere darstellt. Die Abschirmeinrichtung verhindert mindestens eine laterale eine Kontamination der Umgebung der Vorrichtung mit Streulicht. Die Abschirmeinrichtung kann nach oben hin offen sein, da ein Austritt von Streulicht nach oben hinsichtlich einer Lichtkontamination benachbart angeordneter Vorrichtungen unkritisch ist und zudem so eine Beobachtung der sich in dem Behälter befinden Zellkulturen ermöglicht wird. Bei Bedarf kann jedoch die Abschirmeinrichtung selbstverständlich als eine vollumfängliche lichtdichte Barriere eingerichtet sein, welche den Behälter komplett umgibt und so jeglichen Lichtaustritt aus der Vorrichtung verhindert.

Die Beleuchtungseinheit der Vorrichtung weist eine Aufnahmefläche bzw. Aufstellfläche für den Behälter auf, welche in einer Öffnung in der Beleuchtungseinheit angeordnet ist und wobei die Leuchtmittel oberhalb der Aufnahmefläche um diese herum und/oder unterhalb der Aufnahmefläche angeordnet sind. Bei der Öffnung kann es sich insbesondere um die Öffnung der oben erwähnten Vertiefung handeln. Die Leuchtmittel können unterhalb der Aufnahmefläche angeordnet sein, und somit im Wesentlichen den Boden des Behälters anleuchten, wobei unter Boden des Behälters im Wesentlichen der untere Teil des Behälters verstanden wird. Die Leuchtmittel können auch zusätzlich oder alternativ oberhalb der Aufnahmefläche um diese herum angeordnet sein, und somit im Wesentlichen die untere Außenwand des Behälters anleuchten. Durch eine Kombination der beiden Anordnungen der Leuchtmittel können beispielsweise mehr Leuchtmittel in einer Vorrichtung untergebracht werden, was zugleich eine vielfältigere Gestaltung der Lichtbedingungen ermöglicht.

Gemäß weiteren Ausführungsformen der Vorrichtung können die Leuchtmittel konzentrisch um den Mittelpunkt der Aufnahmefläche angeordnet sind und bevorzugt derart ausgerichtet sind, dass die Normale zu ihrer Lichtabstrahlfläche im Wesentlichen senkrecht bezüglich der sich vor der Lichtabstrahlfläche befindenden Außenwand des auf die Aufnahmefläche angeordneten Behälters ausgerichtet ist. Durch eine solche Anordnung treffen die von der Lichtabstrahlfläche ausgehenden Lichtstrahlen größtenteils senkrecht auf die Außenwand des Behälters, wodurch Reflexionsverluste minimiert werden können. Hierbei wird angenommen, dass die Leuchtmittel gerichtetes Licht abstrahlen, welches seine höchste Intensität entlang der Normalen zur Lichtabstrahlfläche erreicht. Um bei variierenden Größen und Formen des Behälters diesen optimal anleuchten zu können, um bei minimalen Reflexionsverlusten möglichst viel Licht in den Behälter zur Suspensionszellkultur einzustrahlen, sind die Leuchtmittel um mindestens eine Achse schwenkbar in der Beleuchtungseinheit angebracht sein. Eine Schwenkachse kann beispielsweise durch zwei Aufhängepunkte eines Leuchtmittels verlaufen und parallel zur Aufstellfläche der Vorrichtung sein.

Gemäß weiteren Ausführungsformen der Vorrichtung kann die Beleuchtungseinheit ein Belüftungssystem mit mindestens einem Belüftungskanal und bevorzugt einem Lüfter aufweisen zum Abführen der Abwärme der Leuchtmittel an die Umgebung der Vorrichtung. Das Belüftungssystem kann folglich aktiv (mit Lüfter) oder passiv (ohne Lüfter) sein. Die Leuchtmittel können Kühlkörper aufweisen, welche von durch den Belüftungskanal strömende Luft durchströmt werden. Die Leuchtmittel können auch selbst zumindest teilweise in dem Belüftungskanal angeordnet sein und durch die darin strömende Luft angeströmt werden. In einer bevorzugten Ausführungsform kann es sich bei dem Belüftungskanal um einen Ringkanal handeln, welcher um die Aufstellfläche des Behälters innerhalb der Beleuchtungsvorrichtung angeordnet ist. Das Belüftungssystem dient nicht nur der Kühlung der Leuchtmittel, sondern kann auch eine ungewollte Aufheizung der Kulturen durch den Betrieb der Leuchtmittel verhindern.

Gemäß weiteren Ausführungsformen der Vorrichtung kann die Beleuchtungseinheit eine Basis und einen darauf aufsetzbaren Deckel aufweisen, wobei die Leuchtmittel samt ihrer Verkabelung in der Basis angeordnet sind. Der Deckel kann mit der Basis durch Schrauben, einen lösbaren Einrastmechanismus oder sonstige Kupplungsmechanismen miteinander verbunden werden. Die Verkabelung, insbesondere die Stromleitungen der Leuchtmittel können in Kanälen verlaufen, die in der Basis der Beleuchtungseinheit ausgebildet sind. Auch der mindestens eine Belüftungskanal kann in der Basis angeordnet sein, wobei die zur Aufnahmefläche führende Öffnung im Deckel vorliegen kann.

Gemäß weiteren Ausführungsformen der Vorrichtung kann die Abschirmeinrichtung entweder als eine Beschichtung auf der Außenwand des Behälters oder als ein Abschirmaufsatz ausgebildet sein, welcher um den Behälter herum angeordnet werden kann und in dieser Position mit der Beleuchtungseinheit koppelbar ist. Im Falle einer Beschichtung als Abschirmeinrichtung kann es sich beispielsweise um einen Lack handeln, welcher die Wand des Behälters lichtdicht macht. Bei dem Abschirmaufsatz kann es sich um einen die Grundform des Behälters nachahmenden Aufsatz handeln, z.B. einen zylindrischen oder quaderförmigen Aufsatz, welcher um die Öffnung angeordnet werden kann. Der Abschirmaufsatz kann mittels zweckmäßiger Befestigungsmittel (z.B. Bajonett-Verschluss oder Rastmechanismus) mit der Beleuchtungsvorrichtung, z.B. an ihrem Deckel, befestigt werden.

Gemäß weiteren Ausführungsformen kann die Vorrichtung ferner ein Fixiermittel aufweisen zum Fixieren des Behälters auf der Beleuchtungseinheit. Das Fixiermittel fixiert die Position des Behälters in der Beleuchtungsvorrichtung und sorgt dafür, dass eine Relativbewegung zwischen Beleuchtungseinheit und Becher ausbleibt, wenn die Vorrichtung in einem Schüttelinkubator aufgestellt wird. Das Fixiermittel kann beispielsweise auf Basis einer Klemmwirkung funktionieren, wobei entsprechende Klemmmittel mit der Oberfläche des Behälters in Kontakt gebracht werden können. Alternativ oder Zusätzlich kann das Fixiermittel einen Saugnapf aufweisen, welcher zum Beispiel Teil der Aufstellfläche der Beleuchtungsvorrichtung sein kann.

Gemäß weiteren Ausführungsformen der Vorrichtung kann der Abschirmaufsatz zugleich als Fixiermittel eingerichtet sein, wobei die Fixierung durch Kontakt mindestens eines Teils der Innenwand des Abschirmaufsatzes mit der Außenwand des Behälters erreicht wird. Dazu kann die Innenwand des Abschirmaufsatzes so geformt sein, dass sie nach Aufsetzen auf die Beleuchtungsvorrichtung zumindest teilweise an der Außenwand des Behälters anliegt und diesen so innerhalb der Vorrichtung fixiert. Es können auch Elemente, beispielsweise in Form von Klemmnoppen, an der Innenwand des Abschirmaufsatzes angeordnet sein, welche bei dessen Aufsetzen auf die Beleuchtungsvorrichtung die Außenwand des Behälters punktuell kontaktieren. Die Elemente können aus einem flexiblen/elastischen Material gefertigt sein und somit eine Fixierung von Behältern unterschiedlichen Größen innerhalb der Vorrichtung ermöglichen.

Gemäß weiteren Ausführungsbeispielen kann es sich bei den Leuchtmitteln um LEDs handeln, wobei jede der LEDs zur Abstrahlung von Licht mit einer andren Farbe eingerichtet ist. Anders ausgedrückt können sich die LEDs hinsichtlich ihres Spektralbereiches unterscheiden. Bei dem Behälter kann es sich bevorzugt um einen Erlenmeyerkolben handeln.

Gemäß weiteren Ausführungsbeispielen kann die Vorrichtung ferner eine Steuerungseinheit zum individuellen Ansteuern der Leuchtmittel aufweisen, welche in der Beleuchtungseinheit angeordnet ist und mit den Lichtmitteln gekoppelt ist. Alternativ kann die Vorrichtung eine Schnittstelle aufweisen, mittels welcher die Leuchtmittel mit einer als externe Einrichtung vorliegenden Steuerungseinheit zum individuellen Ansteuern der Leuchtmittel koppelbar sind. Die Steuerungseinheit kann beispielsweise einen integrierten Schaltkreis oder eine programmierbare logische Schaltung aufweisen, welche mittels einer Benutzerschnittstelle, z.B. eines Tastfeldes samt Anzeige oder einer tastempfindlichen Anzeige, bedient werden kann. Bei der externen Steuerungseinheit kann es sich um ein Softwaremodul handeln, welches auf Computer ausführbar ist. In jedem Fall kann die Ansteuerung der Leuchtmittel mittels der (internen oder externen) Steuerungseinheit auf Basis des digitalen DMX-Steuerungsprotokolls erfolgen. Im Falle einer bevorzugten Ausführungsform, bei welcher die Leuchtmittel als LEDs ausgebildet sind, kann jede LED über einen Dimmer angeschlossen und mit Strom versorgt werden. Die Stromversorgung kann von extern über ein Kabel oder per in der Beleuchtungsvorrichtung eingebautem Akku erfolgen. Die Einstellung der Beleuchtungsintensitäten sowie die Einschaltzeiten der LEDs können mittels eines DMX-Controller als externer oder interner Steuerungseinheit und einer entsprechendes Bedienprogramm (z.B. ecue Lighting Application Suite als Steuersoftware) realisiert werden. Über das Bedienprogramm kann die Intensität und der Einschaltzeitpunkt jeder einzelnen in der Vorrichtung angeordneten LED gesteuert werden. Die beispielhaft genannte Steuersoftware ist bezüglich der Anzahl der Steuerbefehle nicht limitiert, so dass auch komplexe Lichtszenarien (z.B. Simulation von Sonnenauf- bzw. Untergang) realisiert werden können. Die beispielhaft genannte Software erlaubt die Ansteuerung von bis zu 2000 individuellen LEDs pro Lizenz, was einen Betrieb von insgesamt 333 Vorrichtungen ausreichend wäre, wenn von sechs verschiedenen LEDs pro Vorrichtung ausgegangen wird. Somit ist auch bei mit größeren Vorrichtungen zur Nutzung größerer Behälter, wie z.B. Erlenmeyerkolben, einhergehende Bedarf, mehr als die beispielhaft sechs genannten LEDs pro Vorrichtung zur verwenden, ausreichend Potenzial hinsichtlich der Steuerungsmöglichkeiten gegeben.

Der im Rahmen dieser Beschreibung verwendete Suspensionszellkultur kann als eine beliebige Zellkultur verstanden werden, bei der im Idealfall alle enthaltenen Zellen einzeln in Suspension vorliegen können. Die Suspensionszellkultur kann phototrop sein und auch Algen umfassen.

### Kurze Beschreibung der Zeichnungen

In Figur 1 ist eine schematische Ansicht eines Ausführungsbeispiels der erfindungsgemäßen Vorrichtung zum Beleuchten von Suspensionskulturen samt Behälter dargestellt.
In Figur 2 ist eine perspektivische Ansicht einer eines Ausführungsbeispiels der erfindungsgemäßen Vorrichtung zum Beleuchten von Suspensionskulturen samt Behälter dargestellt.
In Figur 3 ist eine Seitenansicht der Basis der in Figur 2 gezeigten Vorrichtung samt Behälter dargestellt.
In Figur 4 ist eine Draufsicht auf die Basis der in Figur 2 gezeigten Vorrichtung dargestellt.
In Figur 5 ist eine Seitenansicht des Deckels der in Figur 2 gezeigten Vorrichtung dargestellt.
In Figur 6 ist eine Draufsicht des in Figur 5 gezeigten Deckels der Vorrichtung dargestellt.
In Figur 7 ist eine Seitenansicht der Abschirmeinrichtung der in Figur 2 gezeigten Vorrichtung dargestellt.

### Detaillierte Beschreibung der Zeichnungen

Der in den Figuren dargestellte Behälter, welcher als Gefäß zur Aufnahme der Suspensionszellkultur dient, stellt einen bevorzugten Teil der hier vorgestellten Erfindung dar, ist jedoch für sie nicht wesentlich. Folglich kann die erfindungsgemäße Vorrichtung den Behälter zur Aufnahme der Suspensionszellkultur aufweisen.

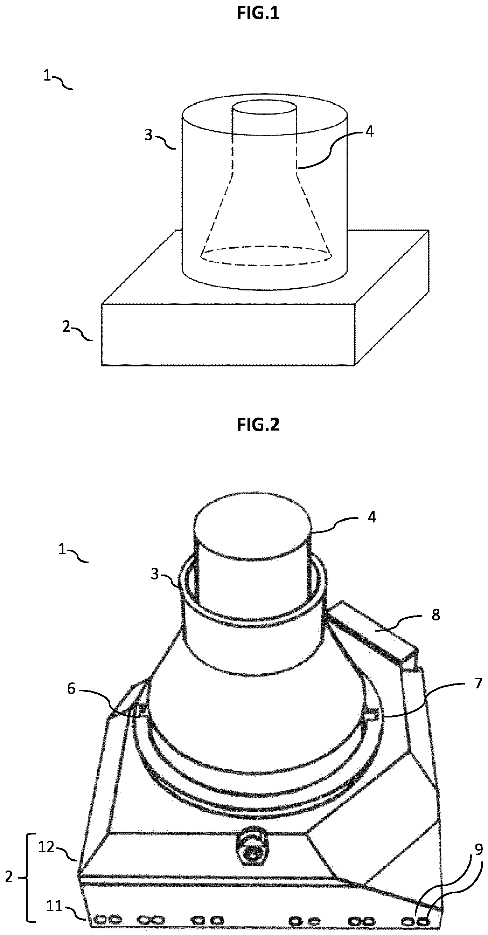

In **Figur 1** ist eine schematische Ansicht eines Ausführungsbeispiels der erfindungsgemäßen Vorrichtung zum Beleuchten von Suspensionskulturen 1 dargestellt. Die Vorrichtung 1 weist eine quaderförmige Beleuchtungseinheit 1 auf, in welcher Leuchtmittel angeordnet sind (in Figur 1 nicht sichtbar). Die Beleuchtungseinheit 1 dient somit als Gehäuse für die Leuchtmittel und stellt zugleich das Bodenstück der Vorrichtung 1 dar. Auf der Beleuchtungseinheit 1 ist der Behälter 4 zur Aufnahme der Suspensionszellkultur angeordnet. Ferner weist die Vorrichtung 1 eine Abschirmeinrichtung 3 auf, welche eingerichtet ist, mindestens lateral eine Kontamination der Umgebung der Vorrichtung 1 mit Streulicht zu verhindern. Die Abschirmeinrichtung liegt hier in Form eines zylinderförmigen Schirms vor, welcher um den Behälter 4 angeordnet ist.

Eine detaillierte perspektivische Draufsicht des in Figur 1 gezeigten Ausführungsbeispiels der erfindungsgemäßen Vorrichtung 1 ist in **Figur 2** dargestellt. Die Beleuchtungseinheit 2 der Vorrichtung weist eine Basis 11 bzw. ein Bodenstück auf und einen Deckel 12 bzw. ein Mittelstück auf, welche getrennt fertigbare Teile sind und miteinander verschraubt oder mittels einer lösbaren Rastverbindung verbunden sind. In Figur 2 ist eine Schraubenaufnahme 10 in Form eines Durchgangslochs gezeigt, so dass die Basis 11 mit dem Deckel 12 verschraubt werden kann. Die Abschirmeinrichtung 3 ist als eine Abschirmhaube ausgebildet und kann mittels Befestigungsmitteln 6, 7, z.B. zwei Bajonett-Verschlüssen, mit dem Deckel 12 der Vorrichtung 1 gekoppelt werden.

Durch den transparent dargestellten Deckel 12 der Vorrichtung 1 ist das Innere der Beleuchtungseinheit 1 sichtbar, in welcher mitunter die Leuchtmittel 5 angeordnet sind, bei denen es sich hier um Leuchtdioden handelt. Die Leuchtmittel 5 sind auf der Basis 11 so angeordnet und leicht nach oben gerichtet, dass sie den Bodenbereich des auf die Beleuchtungseinheit 2 gestellten Behälters 4 anleuchten. Ferner sind die Kabelschächte 9 bzw. Kanäle dargestellt, durch die hindurch die Leuchtmittel 5 ankontaktiert werden können. Die Beleuchtungseinheit 2 weist eine Luftöffnung 8 auf, durch welche Luft in den oder aus dem daran fluidmechanisch gekoppelten Belüftungskanal eingebracht bzw. hinausbefördert werden kann. Es kann zusätzlich ein Lüfter bereitgestellt werden, welcher beispielsweise in der Belüftungsöffnung 8 angeordnet sein kann.

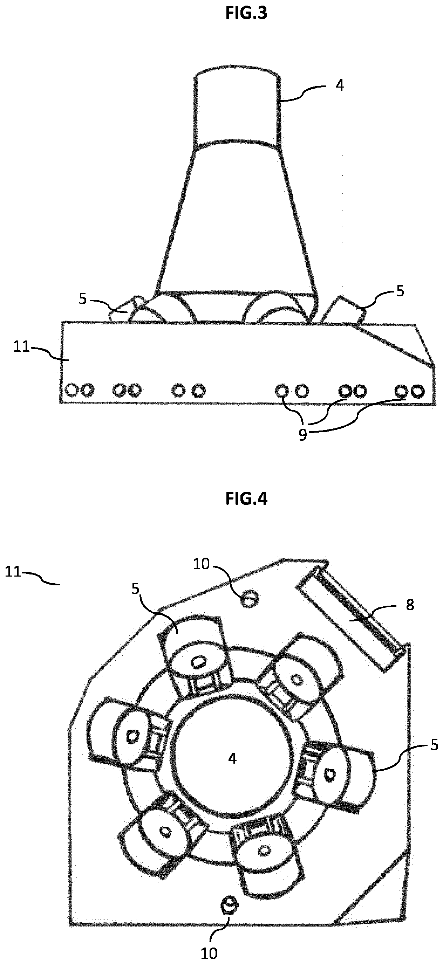

In **Figur 3** ist eine Seitenansicht der in Figur 2 gezeigten Vorrichtung 1 dargestellt, wobei hier nur die Basis 11 und der darauf angeordnete Behälter 4 zu sehen sind, d.h. ohne an die Basis 11 gekoppelten Deckel 12 und ohne an den Deckel 12 gekoppelte Abschirmeinrichtung 3. Eine Draufsicht der Basis 11 ist in **Figur 4** gezeigt. Wie den beiden Figuren entnommen werden kann, sind die Leuchtmittel 5 in äquidistanten Abständen zueinander um die Aufstellfläche (Standfläche) für den Behälter 4 angeordnet. Bei Bedarf kann der Behälter 4 zugleich mit allen Leuchtmitteln 5 angeleuchtet/angestrahlt werden. Durch eine symmetrische Anordnung der Leuchtmittel 5 um den Behälter 4 können die Beleuchtungsbedingungen unter den verschiedenen Leuchtmitteln 5 homogenisiert werden, so dass etwa die geometrischen Reflexionsbedingungen des auf die Außenwand des Behälters 4 treffenden Lichts für alle Leuchtmittel 5 gleich sind. In der in Figur 4 gezeigten Ansicht ist der Belüftungskanal 13 gut sichtbar, welcher ringförmig um die Aufstellfläche des Behälters 4 auf bzw. in der Beleuchtungseinheit 2 angeordnet ist. Der Belüftungskanal 13 ist mit der Luftöffnung 8 gekoppelt und kann zur Umverteilung der Luft im Inneren der Beleuchtungseinheit 2 verwendet werden. So kann die Luft beispielsweise durch einen ersten Verbindungskanal 14 zwischen Luftöffnung 8 und Belüftungskanal 13 in diesen von außen hineinbefördert werden (beispielsweise mittels eines in der Luftöffnung 8 angeordneten Lüfters) und durch einen zweiten Verbindungskanal 15 zwischen Luftöffnung 8 und Belüftungskanal 13 aus diesem wieder an die Umgebung der Vorrichtung 1 abgegeben werden. Auf dem Strömungsweg zwischen den beiden Verbindungskanälen 14, 15 kann die an den Leuchtmitteln 5 vorbei strömende Luft deren Abwärme aufnehmen. Dadurch kann die produzierte Abwärme abgeführt werden und es kann so eine potentielle Erwärmung des Inhalts des Behälters 2 vermieden werden.

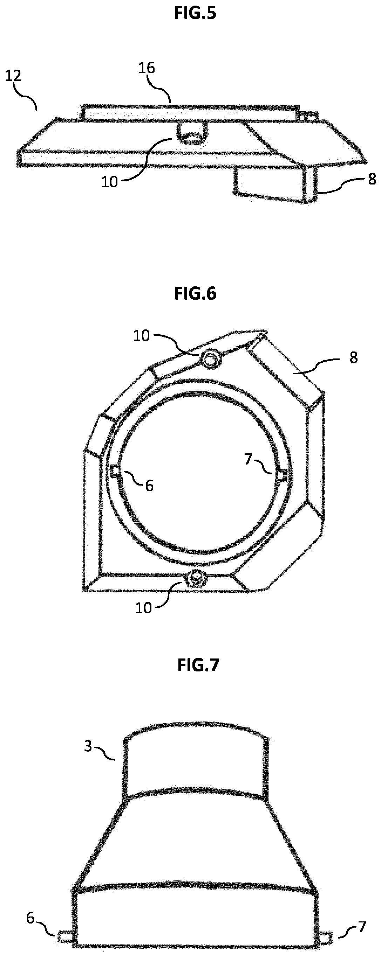

In **Figur 5** ist eine Seitenansicht und in **Figur 6** eine Draufsicht des Deckels 12 der in Figur 2 gezeigten Vorrichtung 1 dargestellt. Durch die im Deckel 12 vorgesehene Öffnung 16 kann der Behälter 2 in die entsprechende in der Vorrichtung 1 vorgesehene Vertiefung eingesetzt werden. In weiteren Ausführungsbeispielen können an der Öffnung Fixierungsmittel angeordnet sein, z.B. radial in die Öffnung einschiebbare Fixierstifte, die den Bodenbereich des in die bzw. auf die Vorrichtung 1 gestellten Behälters 2 ankontaktieren. Dadurch kann eine zusätzliche Fixierung des Behälters 2 erreicht werden.

In **Figur 7** ist eine Seitenansicht der Abschirmeinrichtung 3 der in Figur 2 gezeigten Vorrichtung 1 dargestellt. Die Abschirmeinrichtung 3 ist zylinderartig ausgebildet und weist am unteren Rand Befestigungsmittel 6, 7auf, mit entsprechenden am Deckel 12 angeordneten Befestigungsteilen 6, 7 zusammenwirken, um eine Befestigung/Fixierung der Abschirmeinrichtung 3 gegenüber der Vorrichtung 1 bewirkt. Wie bereits beschreiben kann die lichtdichte Abschirmeinrichtung 3 als Verschlussdeckel in die Vorrichtung 1 bzw. in den Deckel 12 der Vorrichtung eingesetzt werden, um den Behälter 2 innerhalb der Vorrichtung 1 zu fixieren, wie auch um Streulichtkontaminationen zu verhindern.

Bei der erfindungsgemäßen Vorrichtung 1 erfolgt die Beleuchtung der Kulturen nicht wie in kommerziell erhältlichen System von oben (z.B. durch ein Beleuchtungssystem in der Decke des Schüttelinkubators), sondern von unten - also vom Boden der Vorrichtung 1 auf den Bodenbereich des Behälters 4. Die Auslegung der Vorrichtung 1 auf eine bodenseitige Beleuchtung hat den weiteren Vorteil, dass alle Leuchtmittel 5 sowie ggf. Lüfter, Steuerungseinheit und die dazugehörige Verkabelung in der Beleuchtungseinheit 2 angeordnet sind. Dadurch hat die Vorrichtung 1 einen insgesamt tief liegenden Schwerpunkt und einen sicheren Stand, insbesondere bei Verwendung im Schüttelinkubator. Zusätzlich kann durch eine bodenseitige Beleuchtung des Behälters 4 ein besserer Lichteintrag in den Behälter 4 zu der Kulturerreicht werden. Im Gegensatz zur Anbringung eines Beleuchtungsmoduls oberhalb eines Behälters 4 werden Abschattungseffekte und Einstrahlungsverluste durch seine Anbringung an der Bodenseite eines Behälters 4 mit verjüngender Form (z.B. Erlenmeyerkolben) minimiert.

Ferner ist durch die Ausbildung der Vorrichtung 1 als geschlossene lichtdichte Einheit möglich, mehrere dieser Systeme parallel in einem Schüttelinkubator zu betreiben und verschiedene Beleuchtungsbedingungen zu realisieren, ohne dass es zu einer Kontamination benachbarter Kulturen mit Streulicht kommt. Die Vorrichtung 1 kann zur Kultivierung von pflanzlichen Suspensionskulturen oder Algen im kleinen Maßstab verwendet werden, wobei die Kulturen beispielsweise in einem 100 ml Erlenmeyerkolben gehandhabt werden können. Für die Ausleuchtung eines 100 ml Erlenmeyerkolben sind, wie in Figur 2 dargestellt, sechs einzelne LEDs ausreichend. Bei Verwendung größerer Behälter 2 kann sowohl die Abmessung der Vorrichtung 1 wie auch die Anzahl der darin verwendeten Leuchtmitteln 5 entsprechend angepasst werden, was durch eine einfache Anpassung des Designs der die Vorrichtung 1 ausbildenden Einheiten realisiert werden kann . Um ein möglich breites Spektrum unterschiedlicher Lichtbedingungen abdecken zu können, werden Leuchtmittel 5 mit verschiedenen Spektralbereichen eingesetzt. Die in Figur 2 gezeigte beispielhafte Vorrichtung kann etwa mit den folgenden LEDs bestückt werden: a) UV: 365 nm, b) Blau: 455 nm, c) Grün: 505 nm, d) Rot: 660 nm, d) Infrarot: 730 nm, f) Weiß (Wellenlängenbereich nicht genauer definiert), wobei die Wellenlängenangaben nur Orientierungswerte darstellen.

Die hierin offenbarte Vorrichtung zum Beleuchten von Suspensionszellkulturen kann als individuell steuerbares, abgeschlossenes und unabhängiges Systemeinheit betrachtet werden, mit dem eine Vielzahl unterschiedlicher Beleuchtungsbedingungen realisiert werden kann. Die Vorrichtung kann in jeden Schüttelinkubator installiert werden und nur die zur Verfügung stehende Stellfläche innerhalb des Inkubators limitiert die Anzahl der Systemeinheiten, die genutzt werden können.

Die erfindungsgemäße Vorrichtung kann beispielsweise mittels additiver Herstellungsverfahren hergestellt werden, z.B. Lasersintern, Pulversintern oder 3D-Druck. Die Vorrichtung kann auch mittels Fused Deposition Modelling (FDM) Verfahrens hergestellt werden, bei dem das Objekt schichtweise aus einem schmelzfähigen Kunststoff (PLA oder ABS) aufgebaut wird. Bei diesem Herstellungsverfahren lassen sich die die Kabelführungen für die Leuchteinheiten wie auch die Belüftungskanäle in einem Arbeitsgang fertigen.

Dadurch können das Design und die Abmessungen der Vorrichtung einfach und unkompliziert angepasst werden, sollte dies aus technischen Gründen (z.B. Abmessungen/Geometrie der Stellfläche im Schüttelinkubator verschiedener Hersteller) notwendig sein. Der modulare Aufbau erlaubt einen einfachen Austausch der Leuchtmittel, sollten Defekte auftreten oder ein anderer Spektralbereich benötigt werden.

Die vorliegende erfindungsgemäße Vorrichtung ist besonders geeignet für die Optimierung von Kultivierungsbedingungen für pflanzliche Zellkulturen und Algen sowie die Optimierung selbiger in Bezug auf die gesteigerte Produktion wertvoller Inhaltsstoffe, wie beispielsweise sekundäre Pflanzenstoffe, in gewöhnlichen Schüttelinkubatoren, die bezüglich ihrer Aufstellfläche nur eine einheitliche Gestaltung der Beleuchtung ermöglichen.

## Patentansprüche

1. Vorrichtung zum Beleuchten von Suspensionszellkulturen, aufweisend:
eine Beleuchtungseinheit (2), welche mindestens zwei Leuchtmittel (5) aufweist und zur Aufnahme eines Behälters (4) mit einer sich darin befindenden Suspensionskultur eingerichtet ist, wobei die Leuchtmittel um mindestens eine Achse schwenkbar in der Beleuchtungseinheit angebracht sind; und
eine Abschirmeinrichtung (3), welche so lichtdicht ausgebildet ist, dass mindestens lateral eine Kontamination der Umgebung der Vorrichtung (1) mit Streulicht verhindert wird;
wobei die Beleuchtungseinheit (2) eine Basis (11) der Vorrichtung darstellt und der Behälter (4) in die Beleuchtungseinheit (2) gestellt wird; und
wobei die Beleuchtungseinheit (2) eine Aufnahmefläche für den Behälter (4) aufweist, welche in einer Öffnung (16) in der Beleuchtungseinheit (2) angeordnet ist und wobei die Leuchtmittel (5) oberhalb der Aufnahmefläche um diese herum und/oder unterhalb der Aufnahmefläche angeordnet sind.

2. Vorrichtung gemäß Anspruch 1, wobei die Leuchtmittel (5) konzentrisch um den Mittelpunkt der Aufnahmefläche angeordnet sind und bevorzugt derart ausgerichtet sind, dass die Normale zu ihrer Lichtabstrahlfläche im Wesentlichen senkrecht bezüglich der sich vor der Lichtabstrahlfläche befindenden Außenwand des auf die Aufnahmefläche angeordneten Behälters (4) ausgerichtet ist.

3. Vorrichtung nach einem der Ansprüche 1 oder 2, wobei die Beleuchtungseinheit (2) ein Belüftungssystem mit mindestens einem Belüftungskanal (13) und bevorzugt einem Lüfter aufweist zum Abführen der Abwärme der Leuchtmittel (5) an die Umgebung der Vorrichtung (1).

4. Vorrichtung gemäß einem der Ansprüche 1 bis 3, wobei die Beleuchtungseinheit (5) eine Basis (11) und einen darauf aufsetzbaren Deckel (12) aufweist, wobei die Leuchtmittel (5) samt ihrer Verkabelung in der Basis (11) angeordnet sind.

5. Vorrichtung gemäß einem der Ansprüche 1 bis 4, wobei die Abschirmeinrichtung (3) entweder als eine Beschichtung auf der Außenwand des Behälters (4) oder als ein Abschirmaufsatz ausgebildet ist, welcher um den Behälter (4) herum angeordnet werden kann und in dieser Position mit der Beleuchtungseinheit (2) koppelbar ist.

6. Vorrichtung gemäß einem der Ansprüche 1 bis 5, ferner ein Fixiermittel aufweisend zum Fixieren des Behälters auf der Beleuchtungseinheit.

7. Vorrichtung gemäß Anspruch 5 und 6, wobei der Abschirmaufsatz zugleich als Fixiermittel eingerichtet ist, wobei die Fixierung durch Kontakt mindestens eines Teils der Innenwand des Abschirmaufsatzes mit der Außenwand des Behälters (4) erreicht wird.

8. Vorrichtung nach einem der Ansprüche 1 bis 7,
wobei es sich bei den Leuchtmitteln (5) um LEDs handelt, wobei jede der LEDs zur Abstrahlung von Licht mit einer andren Farbe eingerichtet ist; und
wobei es sich bei dem Behälter (4) bevorzugt um einen Erlenmeyerkolben handelt.

9. Vorrichtung nach einem der Ansprüche 1 bis 8, ferner aufweisend:
eine Steuerungseinheit zum individuellen Ansteuern der Leuchtmittel (5), welche in der Beleuchtungseinheit (2) angeordnet ist und mit den Lichtmitteln (5) gekoppelt ist; oder
Schnittstelle, mittels welcher die Leuchtmittel (5) mit einer als externe Einrichtung vorliegenden Steuerungseinheit zum individuellen Ansteuern der Leuchtmittel (5) koppelbar sind.

## Claims

1. Apparatus for illuminating suspension cell cultures, comprising:
an illumination unit (2), which has at least two illuminating means (5) and is configured to receive a container (4) with a suspension culture located therein, the illuminating means being mounted in the illumination unit so as to be pivotable about at least one axis; and
a shielding device (3), which is designed to be light-tight such that at least lateral contamination of the surroundings of the device (1) with stray light is prevented;
wherein the illumination unit (2) constitutes a base (11) of the device and the container (4) is placed in the illumination unit (2); and
wherein the illumination unit (2) comprises a receiving surface for the container (4), which is arranged in an opening (16) in the illumination unit (2) and wherein the illuminating means (5) are arranged above the receiving surface around it and/or below the receiving surface.

2. Apparatus according to claim 1, wherein the illuminating means (5) are arranged concentrically around the center of the receiving surface and are preferably aligned in such a way that the normal to their light-emitting surface is aligned substantially perpendicularly with respect to the outer wall, located in front of the light-emitting surface, of the container (4) arranged on the receiving surface.

3. Apparatus according to any one of claims 1 or 2, wherein the illumination unit (2) comprises a ventilation system with at least one ventilation channel (13) and preferably a fan for dissipating the waste heat of the lighting means (5) to the environment of the apparatus (1).

4. Apparatus according to any one of claims 1 to 3, wherein the illumination unit (5) comprises a base (11) and a cover (12) which can be placed thereon, wherein the illumination means (5) together with their wiring are arranged in the base (11).

5. Apparatus according to any one of claims 1 to 4, wherein the shielding device (3) is formed either as a coating on the outer wall of the container (4) or as a shielding attachment, which can be arranged around the container (4) and in this position can be coupled to the illumination unit (2).

6. Apparatus according to any one of claims 1 to 5, further comprising fixing means for fixing the container on the illumination unit.

7. Apparatus according to claims 5 and 6, wherein the shield attachment is at the same time configured as fixing means, wherein the fixing is achieved by contact of at least a part of the inner wall of the shield attachment with the outer wall of the container (4).

8. Apparatus according to any one of claims 1 to 7,
wherein said illuminating means (5) are LEDs, each of said LEDs being adapted to emit light of a different color; and
wherein the container (4) is preferably an Erlenmeyer flask.

9. Apparatus according to any one of claims 1 to 8, further comprising:
a control unit for individually driving the lighting means (5), which is arranged in the lighting unit (2) and is coupled to the lighting means (5); or
an interface, by means of which the lighting means (5) can be coupled to a control unit, present as an external device, for individually controlling the lighting means (5).

## Revendications

1. Dispositif pour éclairer des cultures de cellules en suspension, comprenant :
une unité d'éclairage (2) qui présente au moins deux moyens d'éclairage (5) et qui est conçue pour recevoir un récipient (4) avec une culture en suspension qui s'y trouve, les moyens d'éclairage étant montés dans l'unité d'éclairage de manière à pouvoir pivoter autour d'au moins un axe ; et
un dispositif de protection (3) qui est conçu de manière étanche à la lumière de telle sorte qu'au moins latéralement, une contamination de l'environnement du dispositif (1) par de la lumière parasite est empêchée ;
dans lequel l'unité d'éclairage (2) constitue une base (11) du dispositif et le récipient (4) est placé dans l'unité d'éclairage (2) ; et
dans lequel l'unité d'éclairage (2) comprend une surface de réception pour le récipient (4), qui est disposée dans une ouverture (16) dans l'unité d'éclairage (2) et dans lequel les moyens d'éclairage (5) sont disposés au-dessus de la surface de réception autour de celle-ci et/ou au-dessous de la surface de réception.

2. Dispositif selon la revendication 1, dans lequel les moyens d'éclairage (5) sont disposés de manière concentrique autour du centre de la surface de réception et sont orientés de préférence de telle sorte que la normale à leur surface d'émission de lumière est orientée sensiblement perpendiculairement par rapport à la paroi extérieure du récipient (4) disposé sur la surface de réception, qui se trouve devant la surface d'émission de lumière.

3. Dispositif selon l'une quelconque des revendications 1 et 2, dans lequel l'unité d'éclairage (2) comporte un système de ventilation avec au moins un canal de ventilation (13) et de préférence un ventilateur pour évacuer la chaleur dissipée des moyens d'éclairage (5) vers l'environnement du dispositif (1).

4. Dispositif selon l'une quelconque des revendications 1 à 3, dans lequel l'unité d'éclairage (5) comprend une base (11) et un couvercle (12) pouvant être posé sur celle-ci, les moyens d'éclairage (5) ainsi que leur câblage étant disposés dans la base (11).

5. Dispositif selon l'une quelconque des revendications 1 à 4, dans lequel le dispositif de protection (3) est configuré soit sous la forme d'un revêtement sur la paroi extérieure du récipient (4), soit sous la forme d'une coiffe de protection qui peut être placée autour du récipient (4) et qui peut être couplée dans cette position à l'unité d'éclairage (2).

6. Dispositif selon l'une quelconque des revendications 1 à 5, comprenant en outre un moyen de fixation pour fixer le récipient sur l'unité d'éclairage.

7. Dispositif selon les revendications 5 et 6, dans lequel la coiffe de protection est également conçue comme moyen de fixation, la fixation étant obtenue par contact d'au moins une partie de la paroi intérieure de la coiffe de protection avec la paroi extérieure du récipient (4).

8. Dispositif selon l'une quelconque des revendications 1 à 7,
dans lequel les moyens d'éclairage (5) consistent en des LED, chacune des LED étant conçue pour émettre de la lumière d'une couleur différente ; et
dans lequel le récipient (4) consiste de préférence en un erlenmeyer.

9. Dispositif selon l'une quelconque des revendications 1 à 8, comprenant en outre :
une unité de commande pour la commande individuelle des moyens d'éclairage (5), qui est disposée dans l'unité d'éclairage (2) et est couplée aux moyens d'éclairage (5) ; ou
une interface au moyen de laquelle les moyens d'éclairage (5) peuvent être couplés à une unité de commande présente en tant que dispositif extérieur pour la commande individuelle des moyens d'éclairage (5).
